# EUROPEAN PATENT APPLICATION

(11) **EP 2 896 365 A1**
(43) Date of publication of application: **22.07.2015**
(21) Application number: 14186979.2
(22) Date of filing: 30.09.2014
(51) Int. Cl.: A61B 6/00, A61B 8/08, A61B 8/00

(54) **Method and apparatus for displaying medical image**

(30) Priority: 20.01.2014 KR 20140006730
(71) Applicant: Samsung Medison Co., Ltd., Gangwon-do 250-870 (KR)
(72) Inventor: Lee, Jin-yong, Gangwon-do (KR); Song, Joo-hyun, Gangwon-do (KR); Kim, Jong - sik, Gangwon-do (KR); Jo, Jae-moon, Gyeonggi-do (KR)
(74) Representative: Frey, Sven Holger

(57) **Abstract**

In a medical image display method, a user receives a still image indicating information about a motion of at least one segment for a predetermined time period and thus may more quickly and accurately recognize the information about a motion of a segment. The medical image display method includes acquiring image data of an object, extracting information about a motion of at least one segment of the object for a predetermined time period, from the image data, and generating and displaying a still image showing information about the motion.

## Description

### CROSS-REFERENCE TO RELATED PATENT APPLICATION

This application claims the benefit of Korean Patent Application No. 10-2014-0006730, filed on January 20, 2014, in the Korean Intellectual Property Office, the disclosure of which is incorporated herein in its entirety by reference.

### BACKGROUND OF THE INVENTION

### 1. Field of the Invention

The present invention relates to a method and apparatus for displaying a medical image, and more particularly, to a method and apparatus for displaying a medical image.

### 2. Description of the Related Art

In the medical image display field, displaying a tissue deformation characteristic such as strain and a strain rate of a regional part of a target object provides a direct and quantitative measurement standard about the ability of muscle that strains and relaxes. The strain signifies a degree of deformation of a regional part of an object and is a value indicating a rate of length decrease or increase from the original length. The strain rate signifies a rate of deformation of the regional part of the object according to time, that is, a deformation velocity.

In particular, displaying strain and a strain rate of muscle as an image in real time may be applied to cardiology. Analyzing the tissue deformation characteristic such as strain and a strain rate of a heart muscle is referred to as heart wall motion analysis. Although a healthy heart does not have a movement disorder at a regional part of a heart wall, that is, a segment forming the heart wall, a heart suffering from a heart disease shows a difference in strain movement between segments of a heart wall, particularly, a difference in strain timing. Thus, whether the segments of a heart wall move with simultaneity is one of the important factors to diagnose a cardiac disease.

In this regard, a method of displaying on a screen an image showing strain and a strain rate of a segment at a predetermined time is used to analyze heart wall motion. However, when only strain and a strain rate of a segment at a predetermined time are provided according to conventional technology, it is difficult to easily identify simultaneity of heart wall motion while a user moves for a predetermined cycle. In other words, the user has difficulty accurately identifying strain timing of each segment of a heart wall by using the strain or strain rate at a predetermined time.

Thus, there is a demand for a method and apparatus for displaying a medical image that provides information about motion so that a user may quickly identify information about motion of a heart wall for a predetermined time period, for example, information about a peak time (when a segment is strained most) of each segment that is an important determination standard for heart wall motion analysis and information about a time difference at the peak time of each segment.

### SUMMARY OF THE INVENTION

The present invention provides a method and apparatus for displaying a medical image which enables an easy and accurate check-up of diseases related to an object by allowing a user to quickly identify information about motion of at least one segment for a predetermined time period.

According to an aspect of the present invention, a method of displaying a medical image includes acquiring image data of an object, extracting information about motion of at least one segment of the object for a predetermined time period, from the image data, and generating and displaying a still image showing information about the motion.

The extracting of the information about the motion may include extracting information about a time point when a characteristic value indicating motion of the at least one segment is one of a minimum value, an intermediate value, a maximum value, and a peak value, as the information about the motion.

The extracting of the information about the motion may include extracting at least one of information about a time point when a characteristic value indicating motion of the at least one segment is a peak value, a peak value of the characteristic value, and a peak value of a change rate of the characteristic value according to time, as the information about the motion for the predetermined time period.

The generating and displaying of the still image may include generating the still image showing the information about the motion by using at least one of a color, a figure, brightness, and a sign.

The generating and displaying of the still image may include generating and displaying an image of the object by using the image data, and displaying information about the motion in an area corresponding to the at least one segment of the displayed image.

The generating and displaying of the still image may include displaying a predetermined image showing the object, and displaying information about the motion in an area corresponding to the at least one segment of the displayed image.

The generating and displaying of the still image may include generating the still image, to which at least one color selected based on the information about the motion is allotted, in an area corresponding to the at least one segment, displaying the still image, and displaying a color bar that indicates the allotted at least one color.

The acquiring of the image data may include acquiring ultrasound image data that includes a plurality of frames of the object, and the extracting of the information about the motion may include selecting a frame from the plurality of frames, and detecting the at least one segment by analyzing the selected frame.

The object may include a heart, the extracting of the information of the motion may include detecting the at least one segment from the image data, and the at least one segment may include a first segment set including a segment of an outer wall of a heart wall of the heart and a segment of an inner wall of the heart wall of the heart, or a second segment set including at least two segments that are divided based on an apex of the heart wall.

The object may include a heart, the extracting of the information about the motion may include extracting information including at least one of information about a time point when strain of the at least one segment is a peak value, a peak strain value, and a peak strain rate, and the generating and displaying the still image may include displaying a first color selected based on one of information about a time point when the strain is a peak value, a peak strain value, and a peak strain rate, in an area corresponding to an outer wall of a heart wall of the heart of the displayed image, and a second color selected based on the other one of the information about the time point when the strain is the peak value, the peak strain value, and the peak strain rate, in an area corresponding to an inner wall of the heart wall of the heart of the displayed image.

According to another aspect of the present invention, an apparatus for displaying a medical image includes a data acquirer for acquiring image data of an object, a processor for extracting information about motion of at least one segment of the object for a predetermined time period, from the image data, and generating a still image showing information about the motion, and a display for displaying the still image.

The processor may extract information about a time point when a characteristic value indicating motion of the at least one segment is one of a minimum value, an intermediate value, a maximum value, and a peak value, as the information about the motion.

The processor may extract at least one of information about a time point when a characteristic value indicating motion of the at least one segment is a peak value, a peak value of the characteristic value, and a peak value of a change rate of the characteristic value according to time, as the information about the motion.

The processor may generate the still image showing the information about the motion by using at least one of a color, a figure, brightness, and a sign.

The processor may generate an image of the object by using the image data, and the display may display the image of the object and display the information about the motion in an area corresponding to the at least one segment of the displayed image.

The display may display a predetermined image showing the object and display information about the motion in an area corresponding to the at least one segment of the displayed image.

The processor may generate the still image, to which at least one color selected based on the information about the motion is allotted, in an area corresponding to the at least one segment, and the display may further display a color bar that indicates the allotted at least one color.

The data acquirer may acquire ultrasound image data that includes a plurality of frames of the object, and the processor may select a frame from the plurality of frames and detect the at least one segment by analyzing the selected frame.

The object may include a heart, the processor detects the at least one segment from the image data, and the at least one segment may include a first segment set including a segment of an outer wall of a heart wall of the heart and a segment of an inner wall of the heart wall of the heart, or a second segment set including at least two segments that are divided based on an apex of the heart wall.

The object may include a heart, the processor may extract information including at least one of information about a time point when strain of the at least one segment is a peak value, a peak strain value, and a peak strain rate, and the display may display a first color selected based on one of information about a time point when the strain is a peak value, a peak strain value, and a peak strain rate, in an area corresponding to an outer wall of a heart wall of the heart of the displayed image, and a second color selected based on the other one of the information about the time point when the strain is the peak value, the peak strain value, and the peak strain rate, in an area corresponding to an inner wall of the heart wall of the heart of the displayed image.

According to another aspect of the present invention, there is provided a computer-readable recording medium having recorded thereon a program for executing the above method.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of a medical image display apparatus according to an embodiment of the present invention;
FIG. 2 is a flowchart of a medical image display method according to an embodiment of the present invention;
FIG. 3 illustrates an example of a still image displayed according to an embodiment of the present invention;
FIG. 4 illustrates an example of a still image displayed according to an embodiment of the present invention;
FIG. 5 illustrates an example of a still image displayed according to an embodiment of the present invention;
FIG. 6 illustrates an example of a still image displayed according to an embodiment of the present invention;
FIG. 7 illustrates an example of a still image displayed according to an embodiment of the present invention; and
FIG. 8 is a block diagram of an ultrasound system adopting the medical image display apparatus according to an embodiment of the present invention.

### DETAILED DESCRIPTION OF THE INVENTION

The terms used in the present specification are used for explaining a specific exemplary embodiment, not limiting the present inventive concept. Thus, the expression of singularity in the present specification includes the expression of plurality unless clearly specified otherwise in context. Unless defined otherwise, all terms used herein including technical or scientific terms have the same meanings as those generally understood by those skilled in the art to which the present inventive concept may pertain. The terms as those defined in generally used dictionaries are construed to have meanings matching that in the context of related technology and, unless clearly defined otherwise, are not construed to be ideally or excessively formal.

In the present specification, when a constituent element "connects" or is "connected" to another constituent element, the constituent element contacts or is connected to the other constituent element not only directly, but also electrically through at least one of other constituent elements interposed therebetween. Also, when a part may "include" a certain constituent element, unless specified otherwise, it may not be construed to exclude another constituent element but may be construed to further include other constituent elements.

In the present specification, an "object" may be a living thing or a non-living thing displayed on an image. Also, the object may be a part of a human and may include organs such as the liver, the heart, the womb, the brain, a breast, the abdomen, etc., or a fetus. Also, the object may include any section of a human body.

Also, in the present specification, a "medical image" may include all images for diagnosis and treatment of a disease by which sectional and volume data of a part of a human body are restored from signals projected to the part, for example, a computed tomography (CT) image, a magnetic resonance imaging (MRI) image, or a positron emission tomography (PET) image, in addition to an ultrasound image.

Also, in the present specification, a "user" may be a medical expert including a doctor, a nurse, a clinical pathologist, a sonographer, or a medical imaging expert, but the present invention is not limited thereto.

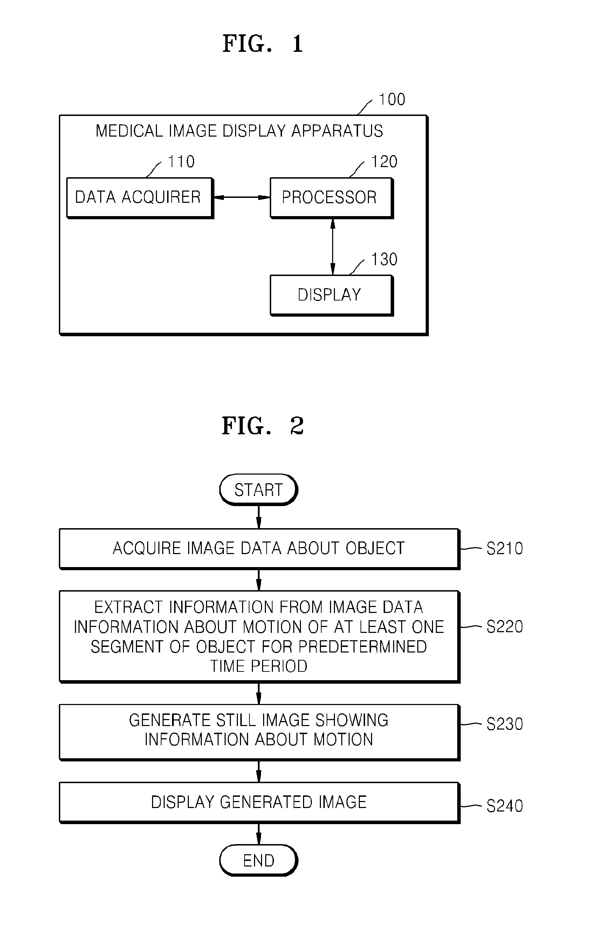

FIG. 1 is a block diagram of a medical image display apparatus 100 according to an embodiment of the present invention. Referring to FIG. 1, the medical image display apparatus 100 includes a data acquirer 110, a processor 120, and a display 130.

The medical image display apparatus 100 according to the present embodiment may be embodied not only in a cart type but also in a portable type. The medical image display apparatus 100 is a device that provides a medical image about an object to a user through a screen of the display 130. Portable medical image display devices may include, for example, a PACS viewer, a smartphone, a laptop computer, a personal digital assistant (PDA), a tablet PC, but the present embodiment is not limited thereto.

The data acquirer 110 acquires image data about an object. The object may include a body part that moves according to a predetermined cycle. For example, the object may be a heart or a heart wall. For example, the data acquirer 110 may acquire ultrasound image data including a plurality of frames that are continuously acquired over time with respect to the object.

In an example, the data acquirer 110 may transmit a predetermined signal to the object, receive a signal that transmits through the object or is reflected from the object, and generate image data by using the received signal.

For example, the data acquirer 110 may include an ultrasonic probe that directly transmits/receives an ultrasonic wave with respect to the object and may generate ultrasound image data.

The ultrasonic probe may transmit an ultrasonic signal to the object and receive an echo signal that is reflected from the object. The ultrasonic probe may transmit an ultrasonic signal to the object according to a driving signal applied to the ultrasonic probe and receive the echo signal.

The ultrasonic probe includes a plurality of transducers that generate ultrasonic waves that are acoustic energy as they vibrate according to applied electric signals. Also, the ultrasonic probe may be connected to a main body of the medical image display apparatus 100 in a wired or wireless manner. The medical image display apparatus 100 may include a plurality of ultrasonic probes according to an embodiment type. According to the present embodiment, the ultrasonic probe may include at least one of a 1 dimension (1 D) probe, a 1.5D probe, a 2D (matrix) probe, and a 3D probe. The data acquirer 110 may further include a data generator that acquires ultrasonic data from an ultrasonic echo signal received from the ultrasonic probe.

In another example, the data acquirer 110 may receive image data about the object from the outside. The data acquirer 110 may receive image data from an external device that is connected to the medical image display apparatus 100 in a wired or wireless manner.

The processor 120 generates a medical image of the object by using the image data acquired by the data acquirer 110. For example, when the data acquirer 110 acquires ultrasound image data, the processor 120 may generate an ultrasound image through a scan conversion process performed on the ultrasonic data acquired by the data acquirer 110.

An ultrasound image may include not only a gray-scale ultrasound image that is obtained by scanning the object according to an A mode (amplitude mode), a B mode (brightness mode), and an M mode (motion mode), but also a Doppler image that indicates motion of the object. The Doppler image may include a blood flow Doppler image (referred to as a color Doppler image) showing blood flow, a tissue Doppler image showing motion of tissue, and a spectral Doppler image showing a movement velocity of the object as a waveform.

Also, when the data acquirer 110 acquires volume data of the object, the processor 120 may generate a 3D medical image by performing a volume rendering process on the volume data. Also, the processor 120 may further generate an elastic image obtained by visualizing a degree of deformation of the object according to pressure and may present various pieces of additional information on the ultrasound image in text or graphics.

The processor 120 extracts information about motion of at least one segment of the object for a predetermined time period from the image data acquired by the data acquirer 110. The object may include at least one segment. The segment may signify a regional part of the object. The motion of the segment may result from deformation of a length of the segment according to strain and relaxation of muscle of the segment.

The medical image display apparatus 100 may analyze an image of the object and thus automatically detect an area corresponding to a segment from the image of the object. For example, when the data acquirer 110 acquires image data including a plurality of frames, the processor 120 may select a predetermined frame of the frames and detect at least one segment. In detail, the medical image display apparatus 100 extracts a contour indicating an entire heart wall by analyzing the selected frame and divides the contour into at least one area by using a preset dividing method. Accordingly, the divided at least one area may be detected as at least one segment. The medical image display apparatus 100 may divide a heart wall according to a cardiology guideline.

Alternatively, the medical image display apparatus 100 may detect an area corresponding to a segment from the image of the object based on a user input. For example, the medical image display apparatus 100 may detect an area set by the user as an area corresponding to the segment with respect to the selected frame. The user input of setting the area corresponding to the segment may be received by a user input device (not shown) that is included in the medical image display apparatus 100. In detail, the medical image display apparatus 100 extracts a contour indicating an entire heart wall based on the user input and divides the contour into at least one area by using the preset dividing method. Accordingly, the divided at least one area may be detected as at least one segment.

The at least one segment of the object may include a first segment set consisting of a segment of an outer wall of a heart wall and a segment of an inner wall of the heart wall. At least one segment of the object may include a second segment set consisting of at least two segments divided with respect to the apex of the heart wall. For example, the medical image display apparatus 100 may detect areas corresponding to 6 segments that are equally divided with respect to the apex of the heart wall, from a sectional image of the heart wall.

The processor 120 may extract information about a time point when a characteristic value indicating motion of the at least one segment for a predetermined time period is a predetermined value, as information about motion. For example, the predetermined time period may include a heart wall strain cycle. The information about a time point when the characteristic value is the predetermined value may include information about a time point when the characteristic value is one of a minimum value, an intermediate value, a maximum value, and a peak value for the predetermined time period.

Also, the processor 120 may extract, as information about motion, at least one of information about a time point when the characteristic value indicating the motion of the at least one segment is a peak value, a peak value of the characteristic value, and a peak value of a change rate of the characteristic value according to time for a predetermined time period. The information about the time point when the characteristic value is the peak value may include information about a time needed for the characteristic value to reach the peak value (time to peak) or information about a time point when the characteristic value is the peak value (time at peak), according to how a standard point is set.

The processor 120 generates a still image showing motion of the at least one segment for a predetermined time period based on the extracted information. The processor 120 may generate a still image that shows information about motion of the segment by using at least one of a color, a figure, brightness, and a sign.

The processor 120 may generate an image showing the object by using image data. For example, an image showing the object may include an ultrasonic B mode image. The processor 120 may include an image showing the object and generate a still image indicating information about motion of the at least one segment. The processor 120 may display information about motion of a segment in an area corresponding to the segment of the image showing the object.

The processor 120 may generate a still image showing information about motion of at least one segment of a predetermined image indicating the object in an area corresponding to the segment. For example, the processor 120 may display an image including a U-shaped diagram that represents an image of a heart wall on the screen and may indicate information about motion of a heart wall on the U-shaped diagram.

The processor 120 may generate a still image to which at least one color that is selected based on the information about motion is allotted, in an area corresponding to the at least one segment of the still image.

The display 130 displays the still image generated by the processor 120 on the screen.

The display 130 may display information about motion of the at least one segment in an area corresponding to the segment of the still image displayed on the screen. For example, the display 130 may display the color selected based on the information about the motion of the at least one segment for the segment.

The display 130 may further display a color bar indicating the at least one color selected based on the information about the motion, with the still image to which at least one color selected based on the information about the motion is allotted.

The display 130 may display an image showing the object and display a first color selected based on one of the information about a time point when the characteristic value is the peak value, the peak value of the characteristic value, and the peak value of a change rate of the characteristic value according to time, in an area corresponding to the outer wall of a heart in the displayed image. Also, the display 130 may display a second color selected based on the other one of the information about a time point when the characteristic value is the peak value, the peak value of the characteristic value, and the peak value of a change rate of the characteristic value according to time, in an area corresponding to the inner wall of a heart in the displayed image.

The medical image display apparatus 100 according to the present embodiment provides a user with a still image indicating information about the motion of the at least one segment for a predetermined time period by using at least one of a color, a figure, brightness, and a sign, so that the user may quickly and accurately identify the information about the motion of the segment.

In the following description, a method of displaying a medical image indicating information about motion of an object by using the medical image display apparatus 100 according to the present embodiment is described in detail with reference to FIG. 2.

FIG. 2 is a flowchart of a medical image display method according to an embodiment of the present invention. Referring to FIG. 2, in operation S210, the medical image display apparatus 100 according to the present embodiment acquires image data about an object. The object may include a part of a human body that regularly moves according to a predetermined cycle. For example, the object may be a heart or a heart wall.

The medical image display apparatus 100 may transmit a predetermined signal to the object, receive a signal that transmits through the object or is reflected from the object, and generate image data by using the received signal. Alternatively, the medical image display apparatus 100 may receive the image data of the object from the outside.

For example, the medical image display apparatus 100 may acquire ultrasound image data including a plurality of frames of the object. The frames of the object may signify a plurality of images that are continuously acquired over time with respect to the object.

In operation S220, the medical image display apparatus 100 according to the present embodiment extracts information about motion of at least one segment of the object for a predetermined time period from the image data.

The medical image display apparatus 100 may detect the at least one segment from the image data. The detection of the segment may signify extracting image data corresponding to the segment from the image data of the object. The medical image display apparatus 100 may acquire information about the motion of the segment by tracking deformation of the segment over time based on the detected at least one segment.

The medical image display apparatus 100 may detect a segment of an outer wall of a heart and a segment of an inner wall of the heart from the image data. Alternatively, the medical image display apparatus 100 may detect at least two segments divided with respect to an apex of a heart wall from the image data. For example, the medical image display apparatus 100 may extract a contour indicating the entire heart wall from the image data and set the apex of the heart wall with respect to the extracted contour. The medical image display apparatus 100 may equally divide the extracted contour with respect to the set apex and detect 6 areas that are obtained by equally dividing the extracted contour, as 6 segments of the heart wall.

First, the medical image display apparatus 100 may select one of the frames acquired for the object. The medical image display apparatus 100 may select the first acquired one of the frames or a frame based on a user's input.

Next, the medical image display apparatus 100 may detect at least one segment by analyzing the selected frame. For example, the medical image display apparatus 100 may detect at least one segment by analyzing the brightness of a gradient of the selected frame. Alternatively, the medical image display apparatus 100 may detect at least one segment based on a user's input for allotting an area corresponding to the segment with respect to the selected frame.

The information about the motion of the segment that the medical image display apparatus 100 extracts from the image data may include information about a time point when a characteristic value indicating the motion of the segment is one of a minimum value, an intermediate value, a maximum value, and a peak value for a predetermined time period. Also, the information about the motion of the segment that the medical image display apparatus 100 extracts from the image data may include at least one of information about a time point when the characteristic value indicating the motion of the segment is a peak value, a peak value of the characteristic value, and a peak value of a change rate of the characteristic value according to time for the predetermined time period.

The characteristic value indicating the motion of the segment may include strain or a strain rate of the segment. For example, the medical image display apparatus 100 may extract from the image data at least one of information about a time point when the strain of the segment of the heart wall is a peak value during a heart wall strain cycle, a peak value of the strain (peak strain value), and a peak value of the strain rate (peak strain rate).

In operation S230, the medical image display apparatus 100 according to the present embodiment generates a still image showing information about the motion extracted in operation S220.

The medical image display apparatus 100 may generate a still image showing the information of motion of at least one segment by using at least one of a color, a figure, contrast, and a sign. The medical image display apparatus 100 may allot at least one color selected based on the information of motion to an area corresponding to the at least one segment of the still image. In this case, the medical image display apparatus 100 may further generate a color bar image showing a relationship between the allotted at least one color and the information of the motion corresponding to the at least one segment.

The medical image display apparatus 100 may generate an image of the object by using the image data acquired in operation S210 and display the information about the motion in the area corresponding to the at least one segment of the generated image. For example, the medical image display apparatus 100 may generate a still image showing the information about the motion of the at least one segment in an ultrasonic B mode image showing the object.

Alternatively, the medical image display apparatus 100 may display the information about the motion in an area corresponding to the at least one segment of a predetermined image showing the object. For example, the medical image display apparatus 100 may generate a still image showing the information about the motion of the at least one segment in a U-shaped diagram that represents the image of the heart wall.

The medical image display apparatus 100 may generate an image showing a heart wall and indicate information of different types of information in an area corresponding to the outer wall of a heart wall and an area corresponding to the inner wall of a heart wall included in the generated image.

For example, the medical image display apparatus 100 may generate an image showing a heart wall and display at least one color in the areas corresponding to the outer wall and the inner wall of the heart wall included in the generated image. The medical image display apparatus 100 may display in the area corresponding to the outer wall of the heart wall the at least one color selected based on one of information about a time point when strain of the at least one segment is a peak vale, a peak value of the strain, a peak value of a strain rate, information about a global peak time, and a first peak time. The medical image display apparatus 100 may display in the area corresponding to the inner wall of the heart wall the at least one color selected based on the other ones of the information about a time point when strain of the at least one segment is a peak vale, the peak value of the strain, the peak value of a strain rate, the information about a global peak time, and the first peak time.

Also, for example, the medical image display apparatus 100 may display the information about the motion of the at least one segment of the outer wall of the heart wall in the area corresponding to the outer wall of the heart wall of the image showing the heart wall. Also, the medical image display apparatus 100 may display the information about the motion of the at least one segment of the inner wall of the heart wall in the area corresponding to the inner wall of the heart wall of the image showing the heart wall.

In operation S240, the medical image display apparatus 100 according to the present embodiment may display the still image generated in operation S230. The medical image display apparatus 100 provides a user with the still image showing the information of the motion of the at least one segment for a predetermined time period by using at least one of a color, a figure, brightness, and a sign so that the user may quickly and accurately identify the information about the motion of the segment.

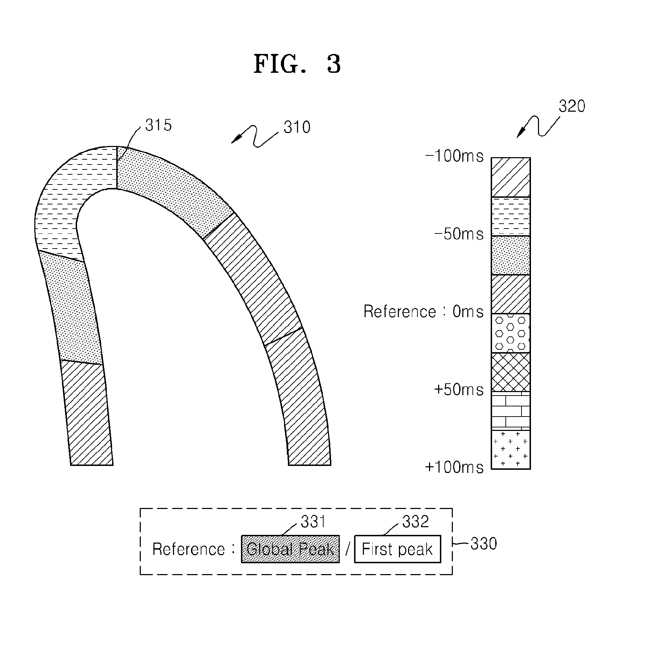

FIGS. 3 to 7 illustrate examples of medical images displayed according to embodiments of the present invention. Although FIGS. 3 to 7 illustrate an example in which the medical image display apparatus 100 acquires image data about a heart wall and displays a still image showing information about motion of a heart wall during a heart wall strain cycle, the present invention is not limited thereto. In particular, FIGS. 3 to 5 and 7 illustrate an example in which the medical image display apparatus 100 displays a still image showing the information about motions of 6 segments included in the heart wall during a heart wall strain cycle.

As illustrated in FIG. 3, the medical image display apparatus 100 may detect a heart wall from the image data acquired with respect to the object and detect the 6 areas of a heart wall that is equally divided with respect to an apex 315 of the heart wall, as segments.

The medical image display apparatus 100 may display a still image 310 that shows information about a time point when strain of each segment is a peak value during a heart wall strain cycle. The medical image display apparatus 100 may acquire information about a change of strain of each segment for a predetermined time period by analyzing image data of a heart wall acquired for the predetermined time period.

The medical image display apparatus 100 may calculate a time difference between a reference time selected within the heartbeat strain cycle and a time point when strain of each segment is a peak value. For example, the reference time may include a global peak time meaning a time point when strain of an entire heart wall is a peak vale and a first peak meaning the earliest time of time points when strain of respective segments are peak values.

As illustrated in FIG. 3, the medical image display apparatus 100 may further display a user interface to select the reference time. For example, the medical image display apparatus 100 may determine to use the global peak time or the first peak time as the reference time based on an input by a user who selects a global peak icon 331 or a first peak icon 332 displayed on the screen.

The medical image display apparatus 100 may select at least one color based on a time difference between the reference time and the time point when strain of each segment is a peak value. The medical image display apparatus 100 may select at least one color corresponding to the time point when strain of each segment is a peak value, based on previously stored mapping information. The medical image display apparatus 100 may further display on the screen a color bar 320 indicating information about the colors selected based on the time difference between the reference time and the time point when strain of each segment is a peak value.

The medical image display apparatus 100 may allot the at least one color selected based on the time point when strain of each segment is a peak value, to the area corresponding to each segment. For example, when the same color is allotted to all segments displayed on the screen, it may signify that the time points when strains of the respective segments are peaks are matched with one another. In other words, it may seen that strain timing of the respective segments of a heart wall are matched with one another.

Accordingly, the user may quickly and accurately identify whether the segments of a heart wall move with simultaneity based on the still image provided by the medical image display apparatus 100 according to the present embodiment.

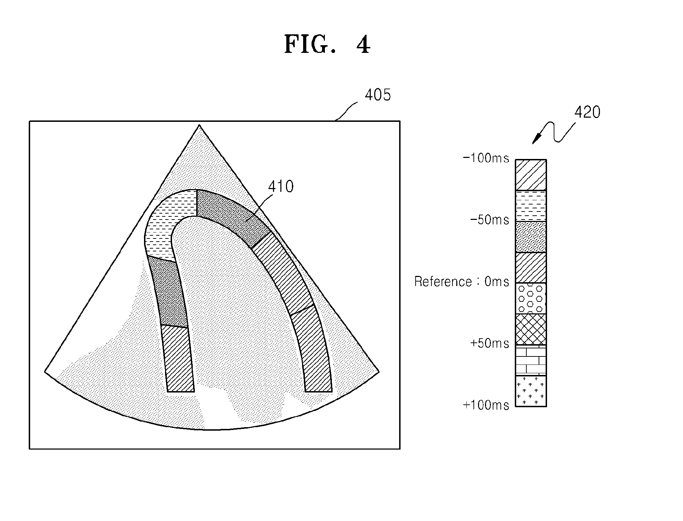

On the other hand, as illustrated in FIG. 4, the medical image display apparatus 100 according to the present embodiment may generate and display an ultrasonic B mode image 405 with respect to the heart wall. The medical image display apparatus 100 may analyze the ultrasonic B mode image 405 and detect an area 410 corresponding to the heart wall and the segments.

The medical image display apparatus 100 may allot a color corresponding to the time point when strain of the segment is a peak value, to the area corresponding to each segment of the ultrasonic B mode image 405. As illustrated in FIG. 4, the medical image display apparatus 100 may further display on the screen a color bar 420 indicating information about the colors selected based on a time difference between the reference time and the time point when strain of each segment is a peak value.

Also, according to the present embodiment, the medical image display apparatus 100 may display on the screen a previously stored image related to the heart wall and allot the at least one color to an area corresponding to the at least one segment of the displayed image. The medical image display apparatus 100 may select a color to be allotted to the area corresponding to the segment based on the information about the motion of each segment.

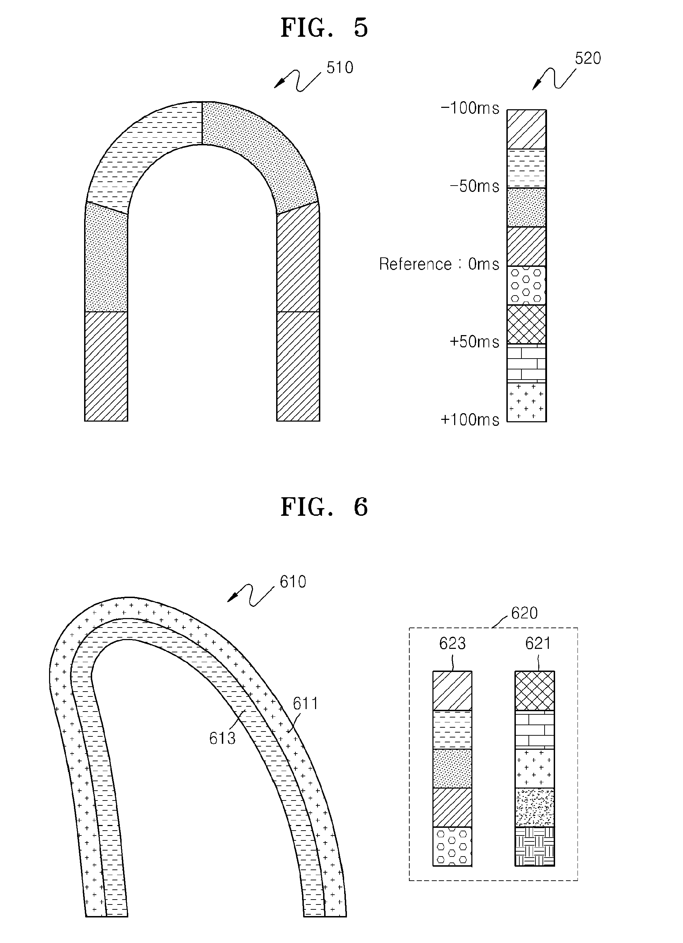

The medical image display apparatus 100 may output on the screen information about the motion of the segment by using an image including a diagram representing the image of the heart wall. As illustrated in FIG. 5, the medical image display apparatus 100 may represent the heart wall by using a reversed U-shaped diagram 510.

As illustrated in FIG. 5, the medical image display apparatus 100 may further display on the screen a color bar 520 indicating information about the colors selected based on the time difference between the reference time and the time point when strain of each segment is a peak value.

The medical image display apparatus 100 according to the present embodiment may display a plurality of types of information on a single screen when outputting information about motion of the at least one segment of the object for a predetermined time period.

As illustrated in FIG. 6, the medical image display apparatus 100 may display an image 610 showing information about motion of the segment for a predetermined time period. The medical image display apparatus 100 may display different types of information in an area 611 corresponding to the outer wall of the heart wall and an area 613 corresponding to the inner wall of the heart wall.

For example, the medical image display apparatus 100 may display in the area 611 corresponding to the outer wall of the heart wall one of information about a time point when strain of the entire heart wall is a peak value, a strain peak value, and a peak value of a strain rate. The medical image display apparatus 100 may display in the area 613 corresponding to the inner wall of the heart wall the other ones of the information about a time point when strain of the entire heart wall is a peak value, the strain peak value, and the peak value of a strain rate.

FIG. 6 illustrates an example in which information about the motion of the heart wall is indicated by using at least one color. The medical image display apparatus 100 may further display a color bar 620 indicating information about the at least one color allotted to the image 610. The color bar 620 provides mapping information about a color that the medical image display apparatus 100 may select based on the information about the motion of the heart wall.

The medical image display apparatus 100 may separately display a color bar 621 providing information about a color allotted to the area 611 corresponding to the outer wall of the heart wall and a color bar 623 providing information about a color allotted to the area 613 corresponding to the inner wall of the heart wall.

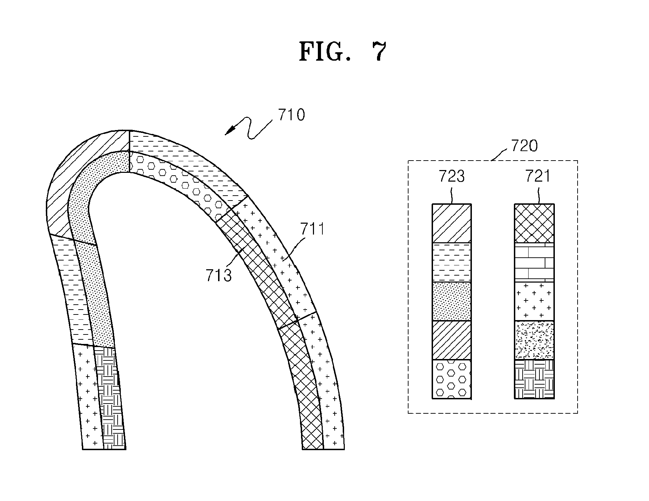

FIG. 6 illustrates a case in which the medical image display apparatus 100 provides information about motion of the entire heart wall. On the other hand, the medical image display apparatus 100, as illustrated in FIG. 7, may provide information about motions of the 6 segments included in the heart wall.

According to the present embodiment, the medical image display apparatus 100 may display a plurality of types of information on a single screen when outputting information about motions of the 6 segments included in the heart wall for a predetermined time period.

As illustrated in FIG. 7, the medical image display apparatus 100 may display an image 710 indicating the information about the motion of the heart wall for a predetermined time period. The medical image display apparatus 100 may display different types of information in an area 711 corresponding to the outer wall of the heart wall and an area 713 corresponding to the inner wall of the heart wall.

For example, the medical image display apparatus 100 may display one of information about the time point when strain of each segment is a peak value, a strain peak value, and a peak value of a strain rate, in the area 711 corresponding to the outer wall of the heart wall. The medical image display apparatus 100 may display the other ones of the information about the time point when strain of each segment is a peak value, a strain peak value, and a peak value of a strain rate, in the area 713 corresponding to the inner wall of the heart wall.

FIG. 7 illustrates an example of displaying information about motion of each segment by using at least one color. The medical image display apparatus 100 may further display a color bar 720 indicating information about the at least one color allotted to the image 710. The color bar 720 provides information about a color that the medical image display apparatus 100 selects based on the information of the motion of each segment.

The medical image display apparatus 100 may separately display a color bar 721 providing information about a color allotted to the area 711 corresponding to the outer wall of the heart wall and a color bar 723 providing information about a color allotted to the area 713 corresponding to the inner wall of the heart wall.

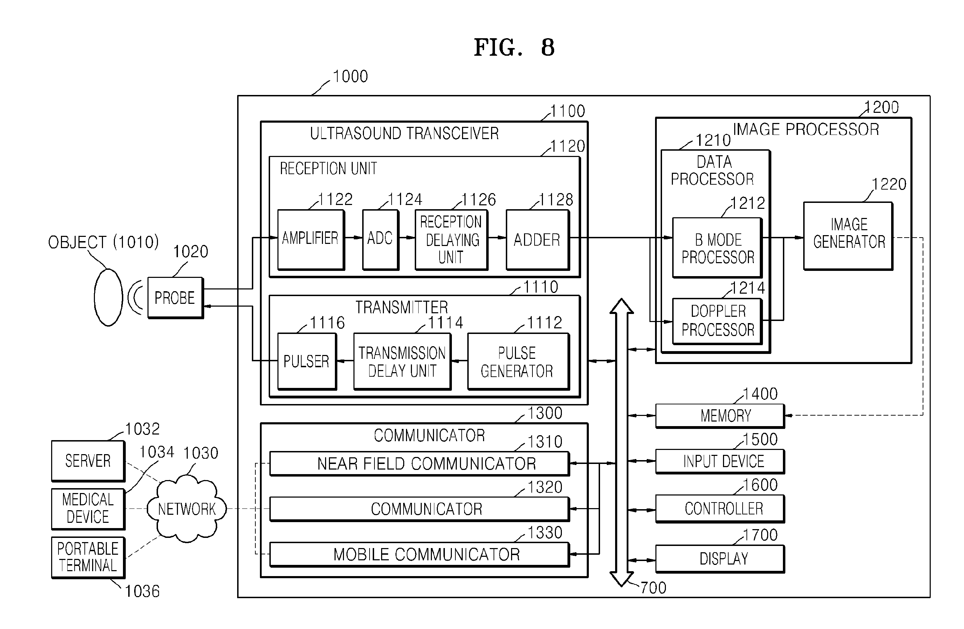

FIG. 8 is a block diagram of an ultrasound system 1000 adopting the medical image display apparatus according to an embodiment of the present invention. When the medical image display apparatus and method according to the present embodiment generates and displays an ultrasound image of the object, the present invention may be applied to the ultrasound system 1000 of FIG. 8.

In other words, the medical image display method according to the present embodiment may be performed by the ultrasound system 1000 of FIG. 8. The medical image display apparatus 100 according to the present embodiment may be included in the ultrasound system 1000 of FIG. 8.

The medical image display apparatus 100 of FIG. 1 may perform part or all the functions performed by the ultrasound system 1000 of FIG. 8. The data acquirer 110 and the processor 120 of FIG. 1 may correspond to a probe 1020, an ultrasound transceiver 1100, and an image processor 1200 of FIG. 8. The display 130 of FIG. 1 may correspond to a display 1700 of FIG. 8.

The ultrasound system 1000 according to the present embodiment may include the probe 1020, the ultrasound transceiver 1100, the image processor 1200, a communicator 1300, a memory 1400, an input device 1500, and a controller 1600, which are connected to one another via a bus 700.

A transmitter 1110 provides a driving signal to the probe 1020. The transmitter 1110 includes a pulse generator 1112, a transmission delay unit 1114, and a pulser 1116. The pulse generator 1112 generates pulses for forming ultrasonic waves to be transmitted, according to a predetermined pulse repetition frequency (PRF). The transmission delay unit 1114 applies a delay time for determining transmission directionality to the pulse. Each pulse to which a delay time is applied corresponds to each of a plurality of piezoelectric vibrators included in the probe 1020. The pulser 1116 applies a driving signal or a driving pulse to the probe 1020 at a timing corresponding to each pulse to which the delay time is applied.

A receiver 1120 generates ultrasound data by processing an echo signal received from the probe 1020. The receiver 1120 may include an amplifier 1122, an analog-to-digital converter (ADC) 1124, a receiving delay unit 1126, and an adder 1128. The amplifier 1122 amplifies the echo signal for each channel. The ADC 1124 performs analog-digital conversion on the amplified echo signal. The receiving delay unit 1126 applies a delay time to determine reception directionality to the digital-converted echo signal. The adder 1128 generates ultrasound data by adding the echo signal processed by the receiving delay unit 1126.

The image processor 1200 generates and displays an ultrasound image through a scan conversion process performed on the ultrasound data generated by the ultrasound transceiver 1100.

A B mode processor 1212 extracts and processes a B mode component from the ultrasound data. An image generator 1220 may generate an ultrasound image in which the strength of a signal is presented in brightness based on the B mode component extracted by the B mode processor 1212.

Likewise, a Doppler processor 1214 may extract a Doppler component from the ultrasound data. The image generator 1220 may generate a Doppler image that expresses a motion of the object in colors or waveforms based on the extracted Doppler component.

The image generator 1220 according to the present embodiment may generate a 3D ultrasound image through a volume rendering process performed on volume data and also generate an elastic image that visualizes a degree of deformation of an object 1010 according to pressure. Furthermore, the image generator 1220 may present various pieces of additional information on the ultrasound image in text or graphics. Also, the generated ultrasound image may be stored in the memory 1400.

The communicator 1300 is connected to a network 1030 in a wired or wireless manner to communicate with an external device or server. The communicator 1300 may exchange data with a server or other medical apparatuses at a hospital that are connected via a picture archiving and communication system (PACS). Also, the communicator 1300 may perform data communication according to the digital imaging and communications in medicine (DICOM) standard.

The communicator 1300 may exchange data related to diagnosis of the object, such as, the ultrasound image, ultrasound data, and Doppler data of the object, and also provide a medical image captured by other medical apparatuses, such as, CT, MRI, and X-ray, via the network 1030. Also, the communicator 1300 may receive information about a diagnosis history of a patient or a treatment schedule from the server and use the received information for diagnosis of the object 1010. Furthermore, the communicator 1300 may perform data communication with not only the server or the other medical apparatuses in the hospital but also with the doctor's or patients portable terminals.

The communicator 1300 may be connected to the network 1030 in a wired or wireless manner and may exchange data with a server 1032, a medical apparatus 1034, or a portable terminal 1036. The communicator 1300 may include one or more constituent elements that enable communication with an external device. The communicator 1300 may include, for example, a near field communicator 1310, a wired communicator 1320, and a mobile communicator 1330.

The near field communicator 1310 signifies a module for near field communication within a predetermined distance. Near field communication technology according to the present embodiment may include wireless LAN, Wi-Fi, Bluetooth, ZigBee, Wi-Fi Direct (WFD), Ultra wideband (UWB), Infrared Data Association (IrDA), Bluetooth Low Energy (BLE), and Near Field Communication (NFC), but the present embodiment is not limited thereto.

The wired communicator 1320 is a module for communication using an electric signal or an optical signal. Wired communication technology may include a pair cable, a coaxial cable, an optical fiber cable, and Ethernet cable.

The mobile communicator 1330 transceives a wireless signal with at least one of a base station, an external terminal, and a server in a mobile communication network. The wireless signal may include a voice call signal, a video call signal, or various types of data according to transceiving of text/multimedia messages.

The memory 1400 stores various pieces of information that are processed by the ultrasound system 1000. For example, the memory 1400 may store medical data related to the diagnosis of the object such as ultrasound data and ultrasound image which are input or output. The memory 1400 may store an algorithm or program that is performed in the ultrasound system 1000.

The memory 1400 may be embodied by a variety of types of storage media such as a flash memory, a hard disk, and EEPROM. Also, the ultrasound system 1000 may run a web storage or a cloud server for performing a storage function of the memory 1400 on the web.

The input device 1500 signifies a device for receiving a user's input of data to control the ultrasound system 1000. The input device 1500 may include a hardware configuration such as a keypad, a mouse, a touch panel, a touch screen, a trackball, and a jog switch, but the present embodiment is not limited thereto. The input device 1500 may further include various input devices such as an electrocardiogram measurement module, a respiration measurement module, a voice recognition sensor, a gesture recognition sensor, a fingerprint recognition sensor, an iris recognition sensor, a depth sensor, and a distance sensor.

The controller 1600 generally controls an operation of the ultrasound system 1000. In other words, the controller 1600 may control operations of the probe 1020, the ultrasound transceiver 1100, the image processor 1200, the communicator 1300, the memory 1400, and the input device 1500, which are illustrated in FIG. 8.

Although some or all of the probe 1020, the ultrasound transceiver 1100, the image processor 1200, the communicator 1300, the memory 1400, the input device 1500, and the controller 1600 may be operated by a software module, the present embodiment is not limited thereto and some of the above elements may be operated by hardware. Also, at least some of the ultrasound transceiver 1100, the image processor 1200, and the communicator 1300 may be included in the controller 1600, but the present embodiment is not limited thereto.

The invention can also be embodied as computer-readable codes on a computer-readable recording medium. The computer-readable recording medium is any data storage device that can store data which can be thereafter read by a computer system. Examples of the computer-readable recording medium include read-only memory (ROM), random-access memory (RAM), CD-ROMs, magnetic tapes, floppy disks, optical data storage devices, etc. The computer-readable recording medium can also be distributed over network-coupled computer systems so that the computer-readable code is stored and executed in a distributed fashion. Also, functional programs, codes, and code segments for accomplishing the present invention can be easily construed by programmers skilled in the art to which the present invention pertains.

While this invention has been particularly shown and described with reference to exemplary embodiments thereof, it will be understood by those skilled in the art that various changes in form and details may be made therein without departing from the spirit and scope of the invention as defined by the appended claims.

## Claims

1. A method of displaying a medical image, the method comprising:
acquiring image data of an object;
extracting information about motion of at least one segment of the object for a predetermined time period, from the image data; and
generating and displaying a still image showing information about the motion.

2. The method of claim 1, wherein the extracting of the information about the motion comprises extracting information about a time point when a characteristic value indicating motion of the at least one segment is one of a minimum value, an intermediate value, a maximum value, and a peak value, as the information about the motion.

3. The method of claim 1, wherein the extracting of the information about the motion comprises extracting at least one of information about a time point when a characteristic value indicating motion of the at least one segment is a peak value, a peak value of the characteristic value, and a peak value of a change rate of the characteristic value according to time, as the information about the motion for the predetermined time period.

4. The method of claim 1, wherein the generating and displaying of the still image comprises generating the still image showing the information about the motion by using at least one of a color, a figure, brightness, and a sign.

5. The method of claim 1, wherein the generating and displaying of the still image comprises:
generating and displaying an image of the object by using the image data; and
displaying information about the motion in an area corresponding to the at least one segment of the displayed image.

6. The method of claim 1, wherein the generating and displaying of the still image comprises:
displaying a predetermined image showing the object; and
displaying information about the motion in an area corresponding to the at least one segment of the displayed image.

7. The method of claim 1, wherein the generating and displaying of the still image comprises:
generating the still image, to which at least one color selected based on the information about the motion is allotted, in an area corresponding to the at least one segment;
displaying the still image; and
displaying a color bar that indicates the allotted at least one color.

8. The method of claim 1, wherein the acquiring of the image data comprises acquiring ultrasound image data that comprises a plurality of frames of the object, and the extracting of the information about the motion comprises:
selecting a frame from the plurality of frames; and
detecting the at least one segment by analyzing the selected frame.

9. The method of claim 1, wherein the object comprises a heart, the extracting of the information of the motion comprises detecting the at least one segment from the image data, and the at least one segment comprises a first segment set comprising a segment of an outer wall of a heart wall of the heart and a segment of an inner wall of the heart wall of the heart, or a second segment set comprising at least two segments that are divided based on an apex of the heart wall.

10. The method of claim 1, wherein the object comprises a heart, the extracting of the information about the motion comprises extracting information comprising at least one of information about a time point when strain of the at least one segment is a peak value, a peak strain value, and a peak strain rate, and the generating and displaying the still image comprises:
displaying a first color selected based on one of information about a time point when the strain is a peak value, a peak strain value, and a peak strain rate, in an area corresponding to an outer wall of a heart wall of the heart of the displayed image, and a second color selected based on the other one of the information about the time point when the strain is the peak value, the peak strain value, and the peak strain rate, in an area corresponding to an inner wall of the heart wall of the heart of the displayed image.

11. An apparatus for displaying a medical image, the apparatus comprising:
a data acquirer for acquiring image data of an object;
a processor for extracting information about motion of at least one segment of the object for a predetermined time period, from the image data, and generating a still image showing information about the motion; and
a display for displaying the still image.

12. The apparatus of claim 11, wherein the processor extracts information about a time point when a characteristic value indicating motion of the at least one segment is one of a minimum value, an intermediate value, a maximum value, and a peak value, as the information about the motion.

13. The apparatus of claim 11, wherein the processor extracts at least one of information about a time point when a characteristic value indicating motion of the at least one segment is a peak value, a peak value of the characteristic value, and a peak value of a change rate of the characteristic value according to time, as the information about the motion.

14. The apparatus of claim 11, wherein the object comprises a heart, the processor extracts information comprising at least one of information about a time point when strain of the at least one segment is a peak value, a peak strain value, and a peak strain rate, and the display displays a first color selected based on one of information about a time point when the strain is a peak value, a peak strain value, and a peak strain rate, in an area corresponding to an outer wall of a heart wall of the heart of the displayed image, and a second color selected based on the other one of the information about the time point when the strain is the peak value, the peak strain value, and the peak strain rate, in an area corresponding to an inner wall of the heart wall of the heart of the displayed image.

15. A computer-readable recording medium having recorded thereon a program for executing the method defined in any of claims 1-10.
